# EUROPEAN PATENT SPECIFICATION

(11) **EP 4 687 750 B1**
(45) Date of publication and mention of the grant of the patent: **28.10.2026**
(21) Application number: 24714179.9
(22) Date of filing: 22.03.2024
(51) Int. Cl.: A61C 19/06, A61N 5/06, A61Q 11/00

(54) **ORAL CARE KIT COMPRISING A LIGHT EMITTING DEVICE AND AN ORAL COMPOSITION**
MUNDPFLEGEKIT, UMFASSEND EINE LICHTEMITTIERENDE VORRICHTUNG UND EINE ORALE ZUSAMMENSETZUNG
KIT DE SOIN BUCCAL COMPRENANT UN DISPOSITIF ÉLECTROLUMINESCENT ET UNE COMPOSITION ORALE

(30) Priority: 29.03.2023 EP 23165137
(43) Date of publication of application: 11.02.2026
(73) Proprietor: Dentaltek Research AG, 8700 Küsnacht (CH)
(72) Inventor: GUAZZO, Pierre, 8700 Küsnacht (CH)
(74) Representative: Carpmaels & Ransford LLP
(86) International application number: PCT/EP2024/057736
(87) International publication number: WO 2024/200246

(56) References cited:
- JP-A- 2012 086 022
- US-A1- 2007 280 888

## Description

### Field of the Invention

The present invention relates to the field of dental devices, specifically to a kit of parts for an oral care implement.

### Background Art

Photodynamic treatment (PDT) was first started in the oral cavity in the mid 1980s. Hematoporphyrins were rapidly replaced by Photofrin and metatetrahydroxyphenylchlorin (mTHPC) as photosensitizers of choice, and over the years these two have been approved by several health authorities for PDT. 5-aminolevulinic acid (ALA) and some dyes (e.g., toluidine and methylene blue) have also been tested. Several different nonthermal lasers have been used and lately light-emitting diodes (LEDs) have been tried. The greatest disadvantage of the common treatment is that the patients are photosensitive for several weeks following systemic administration of the photosensitizer. The bactericidal effect of PDT has also been tested on oral plaque, but little clinical work has been performed so far. Instead of mechanical cleaning or antibiotic therapy, PDT may also play a role in dental diseases.

In professional dentistry, professional oral hygiene is needed in the context of maintenance or as pre-, peri- and post-operative prevention. Today this need is covered by mechanical instruments, e.g., rotational instruments using professional toothpaste, airflow applications, sonic and ultrasonic devices. The drawbacks of this methods may be abrasions which may compromise tooth integrity and soft tissue, difficulties to access all area of the oral cavity, time and skills needed for the application of such methods, as well as the costs of such developed devices.

In addition, chemical agents such as chlorhexidine (CHX) are used primarily in the operative and therapeutic care. Known side effects of CHX are *inter alia* taste disorder, brown staining of teeth, mucosa and tongue, and sometimes desquamation of epithelial cells.

Curcumin is a plant - derived polyphenolic active substance with broad - spectrum antibacterial properties. Curcumin blocks bacterial growth owing to its structural characteristics and the generation of anti - oxidation products. Curcumin can inhibit bacterial virulence factors, inhibit bacterial biofilm formation and prevent bacterial adhesion to host receptors through the bacterial quorum sensing regulation system. As a photosensitizer, curcumin acts under blue light irradiation to induce phototoxicity and inhibit bacterial growth. Moreover, it can exert a synergistic antibacterial effect with other antibacterial substances..

Thus, there is still a medical need for the individual to use such oral hygiene tools such as tooth brush, toothpaste and interdental appliances. However, these tools are not used consistently due to several reasons, e.g., time constrains and frequency needed. In particular for elderly person there is a strong recommendation to use such tools for their daily care. But this recommendation is often neglected due to the sometimes-complicated tools and skills needed by the person or care taking personnel.

The use of electromagnetic radiation in form of light for antimicrobial treatment of the oral cavity is known in the art. For example, WO2020084199 discloses a method of treating biological surfaces with electromagnetic radiation in the form of light of two different energy levels. Specifically, biological surfaces are any surfaces which are subjected to biological contamination caused by or formed by microorganisms, e.g., dental infections caused by dental plaque. The treatment may be combined with an antimicrobial substance, e.g., chlorhexidine to target biofilms for oral disinfection. In order to successfully inactivate biofilms, a combination of high energy photons and low energy photons must be employed.

Further, toothbrushes which emit light are also known, e.g., as described in US2016038762 or WO2021000612.

WO2021034905 discloses a light emitting oscillating toothbrush including a handle with a motor and a light source located in the proximal end. A brush head is in the distal end of the toothbrush, and a drive shaft conveys kinetic energy from the motor to the brush head. The drive shaft includes a light guide from the light source to the distal end and contains two layers of an optical medium of differing refractive indices to enable TIR within the light guide. The brush has a tuft plate having bristles and is made of a polymer such that the water contact angle of the tuft plate is less than 90 degrees and is at least partially transparent. The handle and the tuft plate have electrical contacts to make a contact sensor and a movement sensor provides movement data. A computing device processes movement and contact data to determine when to activate the light.

US2007/280888A1 discloses a dental-plaque detection system and a method that allows dental plaque detection. The dental-plaque detection method includes attaching a dental-plaque staining agent, which contains at least one selected from yellow pigment of beni-koji, turmeric extracts, and curcumin onto an object in the oral cavity; and thereafter irradiating light having a wavelength within a range of 250 to 500 nm onto the object.

JP2012086022A discloses a sensor-responsive toothbrush in which one or more sensors are used for the purpose of detecting a specific condition in the oral cavity or the presence of a specific drug, and can provide one or more reactive outputs .

But it is still challenging to employ daily dental care which is comparable to professional treatment. Therefore, there is still the need for oral hygiene tools with improved properties for daily personal dental care which are less dependent on manual skills, less time consuming and do not only address the surface of the teeth but also the entire oral cavity.

### Summary of invention

It is the object of the present invention to provide oral hygiene tools with improved properties for daily personal dental care or professional dental care. The object is solved by the subject matter of claims 1 and 13.

According to the invention, there is provided a kit of parts for an oral care implement for photo disinfection of the oral cavity, the kit of parts which comprises a light emitting device, and an oral composition comprising curcumin, a curcuminoid, a curcumin derivative, or hypericin, wherein said curcumin, said curcuminoid, said curcumin derivate, or said hypericin is associated with polyvinyl pyrrolidone.

A further embodiment relates to the kit of parts as described herein, wherein said light emitting device comprises a handle, a head, and a light source contained in said head.

A further embodiment relates to the kit of parts as described herein, wherein said head is configured as an angular, oval, or rounded head, as a tray or as a bite splint.

According to one embodiment of the invention, the head is configured to emote the light within all directions to the oral cavity. The light output head may comprise a single light source or multiple light sources. The light output head optionally further comprises a diffuser.

According to one embodiment of the invention, the light may be produced by one or several light-emitting diodes. Optionally, light with high intensity is provided by the light source.

According to one embodiment of the invention the oral composition comprising curcumin, curcuminoids, or a derivative thereof is a fluid, a paste or a gel. The light activatable substance may optionally be an antimicrobial compound.

Further described herein is a method for disinfection of the oral cavity, the method comprising applying a light activatable substance to the oral cavity, irradiating the oral cavity with light provided by a light emitting device at wavelength absorbed by the light activatable substance. The light emitting device may comprise a handle, a head, and a light source contained in said head.

The head is configured as an angular, oval, or rounded head, as a tray or as a bite splint, or is configured as a tray or as a bite splint which comprise several light sources. Optionally, the tray or bite splint is made of translucent material.

### Brief description of drawings

Fig. 1 depicts a dental device with multiple light sources.
Fig. 2 shows a dental device with a single light source and a diffuser.
Fig. 3 shows a dental device similar to a toothbrush.
Fig. 4 shows a dental device configured as a tray.
Fig. 5A and 5B show stained areas at the tartar around the gum line, as well as in between the teeth.
Fig. 6: Depicts the results of the first pilot trial.
Fig. 7: Depicts the results of the second pilot trial.
Fig. 8: Depicts the results of the third pilot trial.
Fig. 9: Depicts the results of the fourth pilot trial.

### Description of Embodiments

The present invention provides a kit of parts for an oral care implement, wherein the kit of parts comprises a light emitting device and an oral composition comprising curcumin, a curcuminoid, a curcumin derivative, or hypericin, wherein said curcumin, said curcuminoid, said curcumin derivate, or said hypericin is associated with polyvinylpyrrolidone.

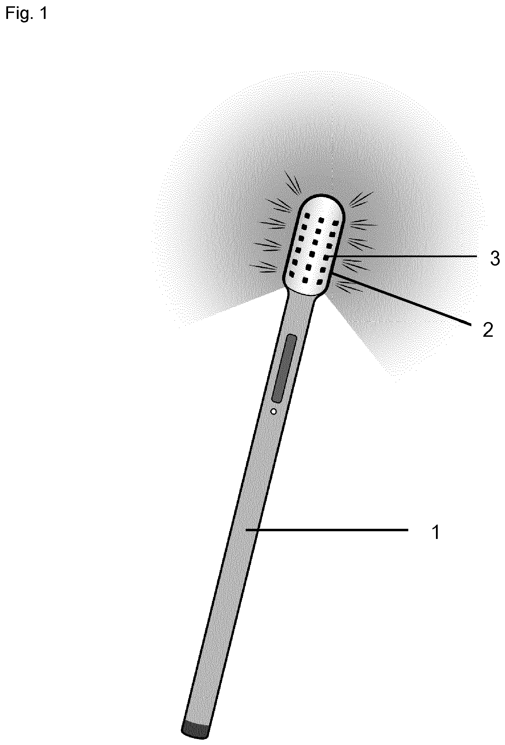

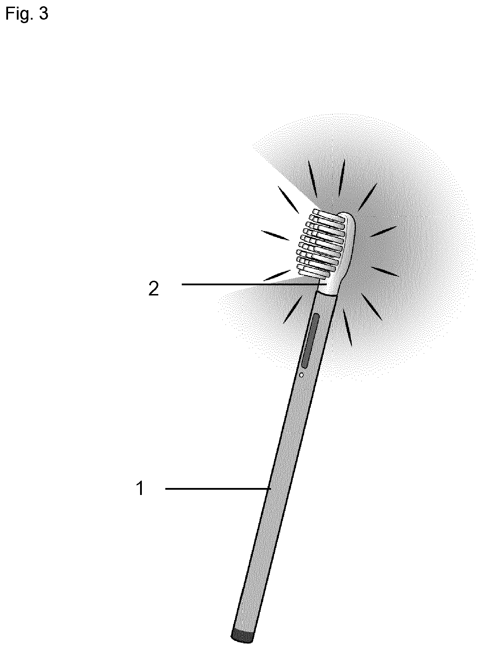

The light emitting device may be comprise a handle (1) and a head (2) configured to emit light (Fig. 1-4). In one embodiment, the head comprises 1, 2, 5, 10, or multiple light sources (3).

Optionally the head may be covered by a diffuser (4). The diffuser may be made of any material that diffuses or scatters light in some manner to transmit soft light. The diffuser is used to break up and distribute light evenly through the oral cavity.

The light source is configured to produce light at a predetermined wavelength and power. The light source comprises a light emitting diode or an array of light emitting diodes.

Suitably, the light source is a filtered white light source. Preferably, the light source is a light emitting diode or array of light emitting diodes. Preferably, the light has a wavelength of from 550 to 690 nm, more preferably from 600nm to 680nm, even more preferably from 625 to 660nm.

Light emitting diodes (LEDs) may be used as the light source. LEDs are available as multiplexed arrays, typically comprising 600 or more individual LEDs, with substantial output powers.

The light emitter may utilize a blue or ultraviolet LED to generate radiation within the 280 to 550 nm, or 650 to 1000 nm using a red or infrared LED. Alternatively, a variety of different light emitters may be utilized in order to provide different radiation characteristics such as different wavelengths or frequency.

In the photodynamic therapy there are many photosensitizer used but when it comes to the oral cavity many of those are not adequate because in the mouth there is a high absorption of the product through the mucosa and the ingestion of a small amount of the photosensitizer cannot be avoided. Furthermore, the taste and the coloring of the photosensitizer play an important role.

The photoactivatable substance may be a radiation reactive agent for inactivating pathogens in the oral cavity, such as for example bacteria.

The concentration of photosensitizer is in the range of 1 to 10.000 µg/mL, or in the range of 1 to 5.000 µg/mL, or in the range of 1 to 2.500 µg/mL, or in the range of 1 to 2.000 µg/mL, or in the range of 1 to 1.000 µg/mL.

It was found that some photosensitizer from natural sources, such as curcumin, curcuminoids, or a derivative thereof, are ideal for the photo disinfection of the oral cavity. Curcumin has anti-inflammatory and antimicrobial properties. Curcumin has the ability to inhibit the production of pro-inflammatory cytokines and enzymes and may help to reduce inflammation and pain in various tissues, including the gums. With regard to its antimicrobial properties, curcumin has the ability to inhibit the growth of various types of bacteria, including those that are associated with dental plaque and gum disease and may help to reduce the severity of oral infections. The low toxicity, limited side effects, availability, low cost, and a full spectrum of beneficial biological properties are among the main advantages of curcumin.

The photoactivatable substance may be a natural photosensitizer compound, such as for example, curcumin. Curcumin ((1E,6E)-1,7-bis-(4-hydroxy-3-methoxyphenyl)-hepta-1,6-diene-3,5-dione) and other curcuminoids constitute the main phytochemicals of Curcuma longa L. (Zingiberaceae family) rhizomes (common name: turmeric). Curcumin is a photosensitizer with phototoxicity and exerts bactericidal effects on various bacteria under blue light excitation. Moreover, curcumin can have synergistic effects with other bacteriostatic substances in combination therapies to increase antibacterial properties.

Curcumin and some of its structurally related analogues ("curcuminoids") may be activated by specific wavelengths of light. Examples of curcuminoids are depicted in the chemical structure below, 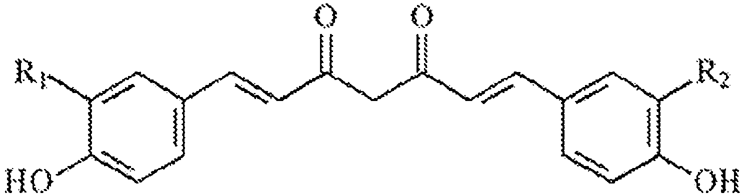 wherein curcumin is represented by R₁ = OCH₃ and R₂ = OCH₃, demethoxycurcumin is represented by R₁ = OCH₃, R₂ = H, and Bisdemethoxycurcumin is represented by R₁ = H and R₂ = H.

Several studies have shown that curcumin has broad-spectrum antibacterial activity and strong biological activity against both Gram-positive and Gram-negative bacteria. Curcumin absorbs blue light (455-460 nm) in the absorption spectrum of 400-500 nm and can be used as an effective natural photosensitizer to promote the success of photodynamic processing.

Blue light-activated curcumin does not exert bacteriostatic effects through direct contact with cells but accomplishes this through the autoxidation mechanism of curcumin. These effects result in the production of intermediates and increase the amounts of oxygen free radicals in cells, thereby disrupting cellular integrity. Reactive oxygen species (ROS) have a short half-life; thus, contact between the photosensitizer and bacterial cells is important. Indeed, the closer the photosensitizer is to the bacterial cell, the more likely there will be a negative effect of ROS on cell integrity. Once bacterial cells are exposed to light, the photosensitizer absorbs the light energy, which is activated to produce ROS, such as hydrogen peroxide, superoxide, and singlet oxygen. ROS then oxidize components of cell membranes, including cholesterol, nitrogen- and sulfurcontaining amino acid residues in proteins, and guanosine in DNA and RNA, leading to bacterial death. Compared to Gram-positive bacteria, Gram-negative bacteria show stronger resistance to the phototoxicity of curcumin.

Tonon et al. (J. Contemp. Dent. Pract. 2015, 16, 1-6 ) combined curcumin and blue light to treat isolated *Streptococcus mutans* (*S. mutans*) colonies obtained from pooled plaque samples of patient with dental caries. The cells were illuminated by a blue LED device for several seconds. It was demonstrated that the curcumin activation with a blue LED was able to photo-inactivate planktonic suspensions of *S. mutans* bacteria.

However, intraoral biofilms are well structured and organized and are hard to penetrate. Physical methods such as mechanical disruption or sonication can physically break apart the biofilm matrix, allowing penetration of antimicrobial agents. Currently the broken biofilm matrix is brushed away and spitted out but usually no antimicrobial agents are applied for the reduction of intraoral biofilms. Toothpaste has a limited antimicrobial effect due to the active ingredients it contains, such as fluoride, triclosan, and essential oils. In addition, some microorganisms may become resistant to the active ingredients in toothpaste over time. This may further reduce the effectiveness of the toothpaste's antimicrobial properties over time. Photodisinfection destroys bacteria, viruses and fungus without harming human cells or causing antimicrobial resistance. Photodisinfection can be used to disrupt the biofilm structure and then eliminate the bacteria in the oral cavity.

Curcumin is the yellow pigment found in the rhizomes of the perennial herb Curcuma longa (turmeric). The bold deep yellow pigments in this turmeric is described to turn teeth as yellow over time. In order to avoid staining of the teeth, curcumin may be subjected to a treatment for providing transparent white and/or a colorless curcumin. White curcumin, may be obtained by catalytic hydrogenation of curcumin. Additionally, the curcumin can be made transparent through the use of ion exchange resin, enzymatic treatment or bleaching it with Hydrogen peroxide in order to make it colorless. However, when exposed to the light emitted by the device, curcumin will be photo-bleached and loses its color anyway. This is also an indication that the disinfection process is sufficient and the yellow stains on the teeth are reduced.

Although, curcumin is fat-soluble, the prime limitation for using curcumin in various application is curcumin's insolubility in aqueous solutions and consequently its poor bioavailability. Heating curcumin in water to boiling for 10 minutes increases the solubility 12-fold. Curcumin solubility can remarkably be increased by associating curcumin with polyvinylpyrrolidone (Front Microbiol. 2018 Jun 15;9:1289). The aqueous solubility of curcumin could be dramatically augmented when curcumin is clicked to galactose (Yadav et al., Sci Rep. 2020 Aug 26;10(1):14204).

Incorporation of mucoadhesive polymers that increase the adhesion of curcumin to the biofilm and oral mucosa may enhances the contact time and effectiveness of curcumin against the bacteria within the biofilm.

Polyvinylpyrrolidone (PVP) can adhere to mucosal surfaces in the oral cavity, potentially increasing the residence time of curcumin on dental biofilms and mucosal tissues. This prolonged contact can enhance the effectiveness of curcumin in targeting oral bacteria within biofilms. PVP can also act as a stabilizer and solubilizer for curcumin, which is typically poorly soluble in water. It can help in forming a stable dispersion of curcumin, thereby improving its bioavailability in the oral environment. Due to its film-forming properties, PVP can create a protective layer that contains curcumin, enabling a sustained release of the active ingredient. This film can adhere to the surfaces of teeth and gums, maintaining the presence of curcumin in the areas where it's needed most. PVP is generally compatible with a wide range of ingredients and can be used in combination with other mucoadhesive polymers, solubility enhancers, or biofilm-penetrating agents, making it a versatile choice in complex formulations.

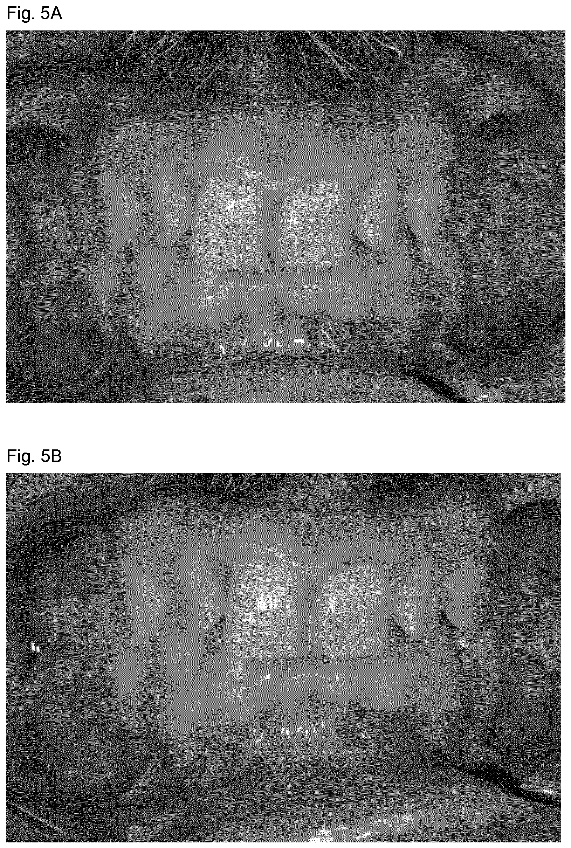

It was found that the oral biofilm has a good wettability to curcumin that has been made water soluble by binding it to polyvinylpyrrolidone, e.g., to PVP-C. Area in the mouth that are more important for a satisfying oral hygiene are also those that quickly absorb this curcumin-PVP. Thus, these critical areas need to be addressed for a targeted photodisinfection in the oral cavity. Both, curcumin and PVP, are already approved as a food additive/stabilizer in the EU and US (E100, E1201) and considered harmless also for oral application. Fig. 5A shows where curcumin-PVP is primarily absorbed after a quick mouth rinse with a composition comprising curcumin-PVP (Fig. 5A, yellow areas) and after washing with water (Fig. 5B). Curcumin-PVP is specifically absorbed on the tartar around the gum line as well as between the teeth. These areas are usually hard to target by the daily teeth hygiene cleaning. The combination of curcumin-PVP as photosensitizing agent in combination with light emitting device for activating the curcumin-PVP not only enables a very targeted effect of the photodisinfection on the area that are more necessary for a good oral health but also reduces the negative effects of light therapy on the oral mucosa or increased tooth sensitivity.

The activation of this photosensitizer needs be done with a very specific light LED array (wave length of 435 ± 10 nm) which will minimize the time of the irradiation. Furthermore, this will reduce the spectrum of the irradiation to a minimum necessary. This reduced time and spectrum enables to reduce the power needed to emit the light considerably and still have a very effective disinfection. This reduces further the negative effects of the radiation and, therefore, makes it possible to use the light to activate the photosensitizer in the oral cavity. In fact, a negative effect of light therapy on oral mucosa and in particular on the gums is gum irritation or inflammation. This can occur if the light source used in the therapy is too intense or the duration of the treatment is too long. The gums may become red, swollen, or tender, and may even bleed in severe cases.

Given those specific requirements of the light needed, intensity and duration to activate curcumin and/or curcumin-PVP a device is needed which emits the light at the areas where the plaque biofilms are specifically located, namely on the tartar around the gum line as well as between the teeth. In fact, using the light emitting device as a tooth brush, will irradiate the area where the biofilm is located. This is also the area which is targeted by conventional and mechanical removal of the plaque.

Additional natural photosensitizer may be used such as for example extracts of ficus racemosa. The extracts are able to reduce the production of inflammatory molecules in human immune cells, and are an anti-inflammatory agent for treating oral mucosal inflammation. The antioxidant activity of ficus racemosa extracts have potential benefits for the oral mucosa and overall oral health. Chlorophyll containing compounds such as dentifrice or mouthwash are associated with reductions in gingival bleeding, plaque accumulation, and bacterial populations in the mouth and effective in reducing oral malodor. Phycobiliproteins are natural pigments found in blue-green algae and certain red algae. Hypericin is found in the St. John's wort plant (Hypericum perforatum), and is a potent photosensitizer with strong antiviral and antidepressant properties. It has been researched for its efficacy in PDT, particularly in treating skin and organ-specific cancers. Phycocyanobilin present in blue-green algae, this pigment is involved in the photosynthetic process and offers potential for photodynamic applications due to its light absorption capabilities. Protoporphyrin IX is a naturally occurring porphyrin, found in many living organisms which plays a role in the biosynthesis of heme and serves as an effective photosensitizer. Pheophorbide a is a chlorophyll breakdown product. Pheophorbide a has been explored for its photosensitizing properties in photodynamic therapy contexts. The photosensitizer compound selected from toluidine blue O, methylene blue, dimethylene blue or azure blue chloride. Psoralens are compounds found in the seeds of Psoralea corylifolia and other plants. Psoralens activate under light exposure and have been used in treating skin disorders. Anthraquinones are naturally occurring in plants like Aloe vera and senna. Some anthraquinone derivatives possess photosensitizing properties relevant to PDT. Bacteriochlorophyll a may be extracted from photosynthetic bacteria. This chlorophyll derivative absorbs light differently than plant chlorophyll, making it useful in less sunlit environments. The active component of saffron, crocetin, absorbs light and generates reactive oxygen species, indicating potential as a photosensitizer. Bilirubin is primarily a heme breakdown product in vertebrates. Bilirubin has been explored for its photosensitizing properties, especially in the context of neonatal jaundice phototherapy.

Within the realm of antimicrobial photodynamic therapy (aPDT), photosensitizers such as toluidine blue O and chlorin e6 have been empirically validated to exhibit substantial antimicrobial efficacy, particularly in eradicating both monospecies and multispecies biofilms. This established effectiveness sets a foundation for exploring alternative photosensitizers that can offer enhanced or comparable antimicrobial properties. Hypericin has inherent photodynamic capabilities, coupled with the advantages of being a natural substance.

The unique combination of hypericin with polyvinylpyrrolidone (PVP) provides a further approach to oral cavity disinfection. This formulation exploits hypericin's photodynamic properties and is significantly enhanced by PVP, which improves the compound's wettability. Such an improvement is crucial for ensuring the uniform distribution and effective adherence of the therapeutic compound across the diverse surfaces within the oral cavity, including teeth, gums, and mucous membranes.

Hypericin offers comprehensive therapeutic benefits, including antioxidant, anti-inflammatory, anticancer, and antimicrobial properties. When activated by light, hypericin induces a potent photodynamic reaction, effectively targeting and eliminating pathogenic microorganisms without disrupting the oral cavity's delicate ecological balance. This property makes the hypericin-PVP formulation an ideal candidate for non-invasive, efficient oral disinfection practices.

Furthermore, the combination with PVP not only enhances the physical application of hypericin but also contributes to a safer and more controlled delivery mechanism within the oral environment, minimizing the risk of irritation or adverse reactions. The inherent non-mutagenic nature of this formulation ensures its safety for regular use, posing no risk of DNA damage or carcinogenic effects.

Incorporating this novel hypericin-PVP formulation into oral health care routines offers a significant advancement in the prevention and treatment of oral infections, providing a powerful, safe, and effective method for disinfecting the oral cavity. This makes it an invaluable tool in both clinical settings and home care, promising a substantial improvement in oral hygiene practices and overall dental health.

The composition may further comprise an agent suited to bleach teeth. Common bleaching compositions may include a peroxide-based material such as hydrogen peroxide, carbamide peroxide, carbamide peroxide, calcium peroxide, sodium percarbonate, perhydrol urea and peroxy-acetic acid.

The composition optionally further comprises a dye, fragrance, flavors, or titanium dioxide.

The composition may be a liquid, paste or gel.

The device according to the invention provides for an additional benefit of an overall disinfection of the mouth. Due to the overall disinfection of the mouth also bad breath caused by microbiota in the oral cavity can be eliminated.

During operation, the composition comprising the photoactivable substance is applied to the oral cavity and radiation having the desired characteristics is generated with the light emitter and transmitted not only to the teeth but to the whole oral cavity.

The composition comprising a photoactivatable substance is applied to the oral cavity similar to the application of a mouth wash. The composition remains in the mouth and then the light emitting device is inserted in the mouth and moved around like a toothbrush in order to irradiate all the parts of the cavity. The photoactivatable substance is activated by the light and inactivates microbes potentially present in the oral cavity. Due to the unique design, also the interdental areas, the oral mucosa and the tongue are reached by the device. Therefore, the device is able to reach areas which are very difficult to accessed by standard mechanical oral hygiene devices. This is achieved by the additional capacity of the device to further distribute the light into small cavities and fissures.

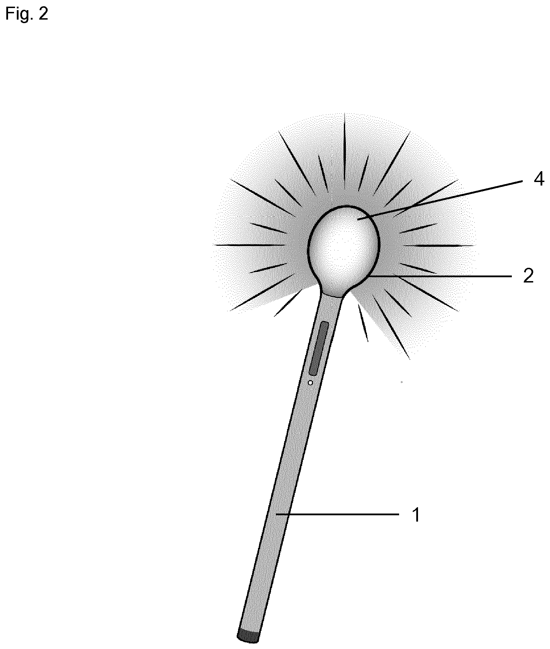

The light emitting device has the property of emitting the light evenly in all direction around the source, similarly to a light bulb. This may be achieved either with multiple light sources (Fig. 1) or through a single light source covered by a diffuser (Fig. 2). It is important that the light is spread everywhere around the area with high intensity in the short range and rapidly decreasing in intensity when move away from the light source. This is a difference from other known devices which try to convey the light in a very focused way to a very specific area or point in the mouth via a light beam.

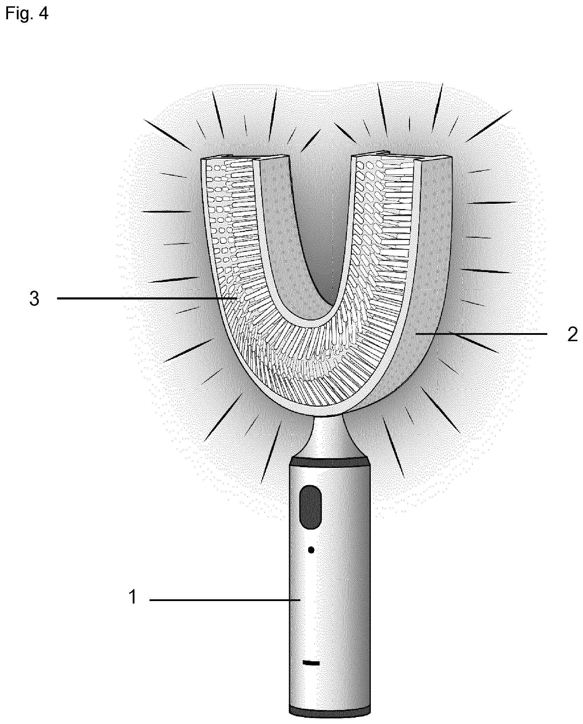

The head of the light emitting device may also be configured as a tray or as a bite splint which comprise several light sources (Fig. 4). According to one embodiment of the invention, the tray or bite splint provides the light and optionally the photoactivatable substance to the teeth or to the denture.

In summary, the combination of curcumin and photodisinfection is quite effective specifically, because the bacteria cannot build resistance to the photodisinfection over time.

### METHODOLOGY

A water-soluble curcumin mouthwash was applied and experiments conducted to assess its effects on oral hygiene, particularly in terms of staining of teeth and gums and biofilm interaction.

The participants rinsed their mouths with a Cur-PVP mouthwash. The mouthwash contained 1mg/ml Cur-PVP.0 Then photographs of the mouth and teeth were taken to observe changes on color and biofilm presence on teeth and gums.

### OBSERVATIONS

The Cur-PVP mouthwash resulted in slightly yellow staining on teeth. After rinsing with water, the effect disappears to a large extent, however, it might bother the user (see).

There was a noticeable staining of the gum color, towards a more reddish color. After rinsing with water, the effect disappears to a weak extent, however, the color of the gums appear healthy and natural, users might experience the effect in a rather positive way.

Specifically in areas where biofilm formation was evident, the yellow staining was very strong. This was interpreted as an effective interaction between Cur-PVP and biofilms. This is a strong finding as Cur-PVP is used in order to reduce the amount of bacteria during oral health care.

### CONCLUSIONS

The water-soluble curcumin mouthwash shows promising results in targeting areas where biofilm forms, which is crucial for effective oral treatment. Staining of the gums can be considered a weak positive outcome. The temporary yellow coloring of teeth, while not ideal aesthetically, fades after rinsing with water to a large extent and does not outweigh the mouthwash's benefits.

### PILOT STUDY

A pilot study was conducted in order to investigate the influence of treating of a supragingival biofilm with a curcumin solution and subsequent radiation with LED.

Curcumin is based on a natural extract of curcuma and is a photoactive molecule. The product is a curcumin complex bound to polyvinylpyrrolidone (Cur-PVP) in order to improve its solubility. The photosensitizer was provided by Planta Natural Products (Planta AG, Vienna, Austria). The product was delivered as a yellow powder, 100 mg Cur-PVP containing 3.2 mg curcumin. The powder was solved in distilled water and desired concentrations were obtained. The product was protected from light up to the time-point of application.

### Experimental setup

Formation *in vitro* of the biofilm with 6 strains typically found in supragingival biofilms: *Candida albicans,* strain ATCC 32032T; *Streptococcus mutans,* strain ATCC 700610; *Streptococcus oralis,* strain OMZ 607 SK248; *Veillonella dispar,* strain ATCC 17748T; *Fusobacterium nucleatum,* strain OMZ 598; and, *Actinomyces oris,* strain OMZ 745.

For the precultures, all strains were transferred onto blood agar plates (Columbia Blood Agar, CBA with 5% defibrillated sheep blood). The blood agar plates were incubated anaerobically at 37° C for 72 h, except for *C. albicans which* was cultivated with 10% CO₂ at 37° C. After transferring to FUM medium with 0.3% glucose and incubating overnight, the strains were stored anaerobically (except for *C. albicans with* 10% CO₂) in fresh FUM medium with 0.3% glucose for 5 hours at 37° C. A processed human saliva pool (diluted pasteurized KOMI donor's pool) was used to condition the 9 mm hydroxyapatite discs (Clarkson Chromatography Products) for at least 4 hours at room temperature, with gentle shaking. Once the pellicle formed, the HA discs were moved to 24-well culture dishes (Thermo Scientific) containing a mixture of processed saliva and FUM medium supplemented with 0.3% glucose. In order to reduce the media, the mixture was held at 37° C for 45 minutes. The OD of each bacterial suspension was adjusted to 1.0 at 550 nm. After preparation of inoculum with the same volume of each bacterial suspension it was added to the reduced medium and then incubated for 16 h at 37° C under anaerobic conditions. Three daily washes were performed on all the discs, with the culture media being replenished every morning. A supplemented FUM media with 0.3% glucose was used for the first 16 hours, then switched to a FUM Media with 0.15% glucose and 0.15% sucrose for the next 24 hours. After 40 h of biofilm growth, biofilms were washed as described and harvested for culture analysis. Colony forming units (CFU) were counted under light microscopy.

### Detailed groups for pilot trials 1-4

### 1. In vitro efficiency of aPDT with Curcumin-PVP on a 6-species biofilm model I

Aims of the project: To evaluate the efficiency of aPDT with curcumin-PVP on a 6-species biofilm model

Null hypothesis: There is no difference of the total CFU when comparing aPDT with Curcumin-PVP with a blue light versus an untreated control Materials to be tested:

### No treatment Control Group

LED Control Group: 4 LEDs 10 seconds light
1min Incubation with 1 mg/ml Curcumin
1min Incubation with 0.01 mg/ml Curcumin + 4 LEDs 10 seconds light
1min Incubation with 0.1 mg/ml Curcumin + 4 LEDs 10 seconds light
1min Incubation with 1 mg/ml Curcumin + 4 LEDs 10 seconds light Experimental procedure: Supragingival Biofilm, 40h, CFU total
Type of the tested microorganisms/biofilm, at which the treatment/intervention is made: CFU total, A. Oris (OMZ 745), V. dispar (OMZ 493), E. nucleatum (OMZ 598), S. mutans (OMZ 918), S. oralis (OMZ 607), C. albicans (OMZ 1134)
Number of treatment groups: 6
Number of repetitions: 1
Number of runs: 1

### 2. In vitro efficiency of aPDT with Curcumin-PVP on a 6-species biofilm model II

Aims of the project: To evaluate the efficiency of aPDT with Curcumin-PVP on a 6-species biofilm model including the effect of incubation time and washing before light application

Null hypothesis: There is no difference of the total CFU when comparing aPDT with Curcumin-PVP with a blue light versus treatment with CHX 0,2% Materials to be tested:

### Control Group

LED Control Group: 4 LEDs 10 seconds light
3 min Incubation with 1 mg/ml Curcumin
2 min Incubation with 1 mg/ml Curcumin + 4 LEDs 10 seconds light
2 min Incubation with 1 mg/ml Curcumin + Wash + 4 LEDs 10 seconds light
3 min Incubation with 1 mg/ml Curcumin + 4 LEDs 10 seconds light
3 min Incubation with 1 mg/ml Curcumin + Wash + 4 LEDs 10 seconds light
2 min application of 0.02% CHXglu
Experimental procedure: Supragingival Biofilm, 40h, CFU total
Type of the tested microorganisms/biofilm, at which the treatment/intervention is made: CFU total, A. Oris (OMZ 745), V. dispar (OMZ 493), E. nucleatum (OMZ 598), S. mutans (OMZ 918), S. oralis (OMZ 607), C. albicans (OMZ 1134)
Number of treatment groups: 6
Number of repetitions: 1
Number of runs: 1

### 3. In vitro efficiency of aPDT with curcumin-PVP on a 6-species biofilm model III

Aims of the project: To evaluate the efficiency of aPDT with curcumin-PVP on a 6-species biofilm model including the effect several applications of the treatment during the 40-hour biofilm growth.

Null hypothesis: There is no difference of the total CFU when comparing aPDT with Curcumin-PVP with a blue light versus treatment with CHX 0,2% Materials to be tested:

### Control Group

LED Control Group: 4 LEDs 10 seconds light at 16h and 40h
LED Control Group: 4 LEDs 10 seconds light at 16h, 24h, 40h
3 min Incubation with 1 mg/ml curcumin at 16h and 40h
3 min Incubation with 1 mg/ml curcumin at 16h, 24h, 40h
3 min Incubation with 1 mg/ml curcumin + 4 LEDs 10 seconds light at 16h and 40h
3 min Incubation with 1 mg/ml curcumin + Wash + 4 LEDs 10 seconds light at 16h, 24h, 40h
2 min application of 0.02% CHXglu at 16h and 40h
2 min application of 0.02% CHXglu at 16h, 24h, 40h
Number of treatment groups: 9
Number of repetitions: 1
Number of runs: 1

### 4. In vitro efficiency of aPDT with curcumin-PVP on a 6-species biofilm model III

Aims of the project: To evaluate the efficiency of aPDT with Curcumin-PVP on a 6-species biofilm model versus methylene blue and to compare the efficiency of a novel light source

Null hypothesis: There is no difference of the total CFU when comparing aPDT with Curcumin-PVP with a blue light versus methylene blue activated with a red light

### Materials to be tested:

### Control Group

LED Control Group: 4 LEDs 10 seconds with 2W (440nm)
LED Control Group: 1 LED 10 seconds with 100W (440nm)
LED Control Group: 1 LED 10 seconds with 5W (660nm)
5 min Incubation with 1 mg/ml Curcumin
5 min Incubation with 1 mg/ml Methylene blue
5 min Incubation with 1 mg/ml Curcumin + 4 LEDs 2W for 10 seconds
5 min Incubation with 1 mg/ml Curcumin + 1 LED 100W for 10 seconds
5 min Incubation with 0.1 mg/ml Methylene blue + 1 LED 5W (660nm) for 10 seconds
Number of treatment groups: 9
Number of repetitions: 2
Number of runs: 1

### Statistical analysis

Data was recorded in a spreadsheet (Microsoft Excel, Microsoft Corporation, Redmond, Washington, USA) and scatterplots were obtained. Due to the low number of specimen during the pilot phase, further statistical analyses were not feasible.

### Results

Except for the last pilot trial, each dot represents a single value. No means or standard deviations are available. From other trials with similar methodology it is known that a factor x1000 in total CFUs will usually provide a significant result if trial is properly powered.

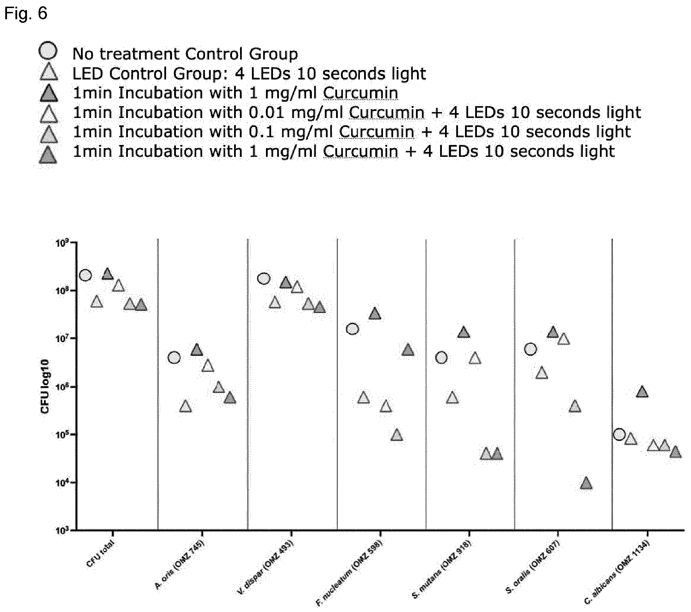

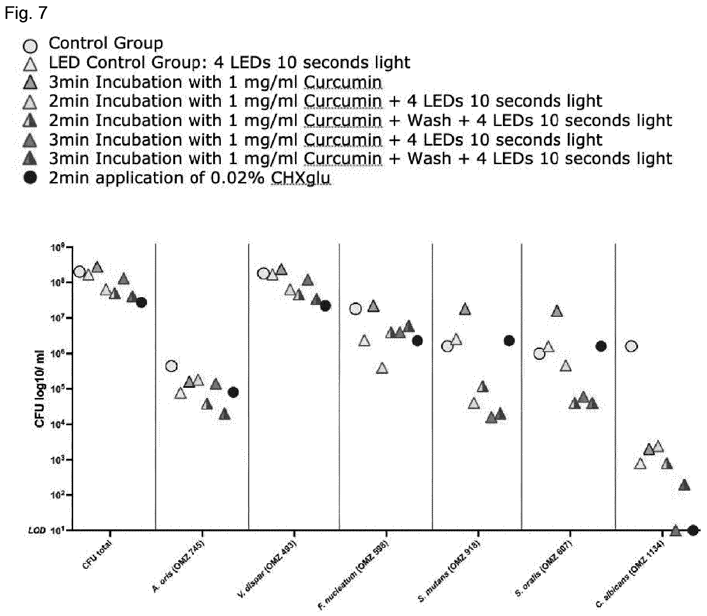

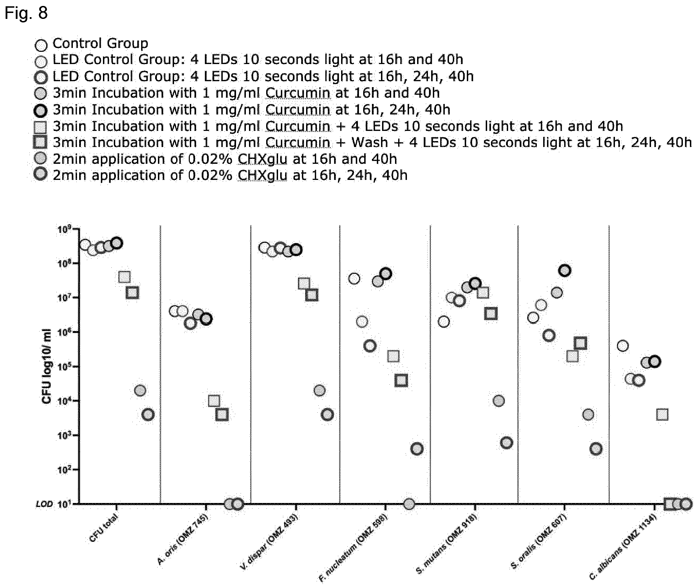

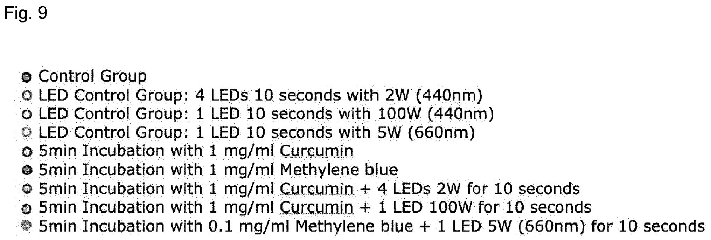

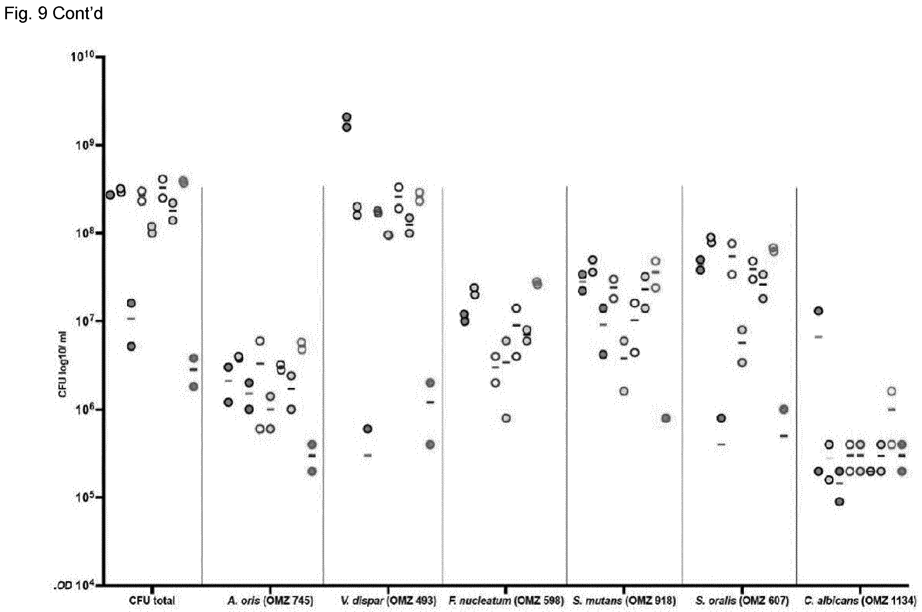

As shown in Fig. 6, the total CFU (log) are all ranging around 10⁸. For the second pilot trial, as shown in Fig. 7, the total CFU (log) are ranging around 10⁷-10⁹. It appears that one of the aPDT groups and chlorhexidine have a strong impact on *C. albicans.* For the third pilot trial, as shown in Fig. 8, the total CFU (log) are ranging around 10⁴ for both chlorhexidine applications. Compared to the control groups, the two aPDT groups where able to reduce the total CFU by 10¹-10². For the fourth pilot trial, as shown in Fig. 9, the total CFU (log) are ranging around 10⁶-10⁷ for both methylene blue applications, with and without light activation.

### Discussion

The present sequence of pilot trials revealed:
i) Antibacterial photodynamic therapy with Cur-PVP was able to target specific bacteria strains that are without strongly affecting the total CFU. Targeted reduction of pathogenic bacteria: Streptococcus mutans, responsible for dental caries and tooth decay (OMZ 918), *Streptococcus oralis,* an early colonizer in the formation of dental plaque (OMZ 607), *Fusobacterium nucleatum,* contributing to the development of the biofilm (OMZ 598);
ii) Repeated applications of the therapy led to a larger effect;
iii) Chlorhexidine and aPDT with methylene blue were more effective in reducing total CFU and less selective in targeting specific bacteria strains;
iv) The natural photosensitizer remains neutral when used alone, suggesting a lower potential for toxicity and side effects. The same when using the blue light alone.

Although the pilot trials do not provide means or standard deviations, the constant levels of the controls, including negative controls with light only and Cur-PVP only, indicate that the data is robust to a certain extent. It further indicates that the experimental setup was meticulously conducted and was able to deliver similar outcomes when repeated.

The incubation time of Cur-PVP, the concentration of Cur-PVP, the washing before light application and different intensities of irradiation were evaluated throughout the four pilot studies. Three applications in 40 hours led to a positive effect.

Another finding is that light only and Cur-PVP only did not have any effect. Still, it appears that only the activation by light led to a reduction of bacteria. This is an important finding for a preventive application, as side effects cannot be tolerated for a therapy that is applied daily. This was not the case with methylene blue. Although aPDT with methylene blue was more effective, methylene blue alone already had a strong effect on the number of bacteria.

## Claims

1. A kit of parts for an oral care implement for photo disinfection of the oral cavity, the kit of parts comprising:
a. a light emitting device, and
b. an oral composition comprising curcumin, a curcuminoid, a curcumin derivative, or hypericin,
wherein said curcumin, said curcuminoid, said curcumin derivative, or said hypericin is associated with polyvinylpyrrolidone.

2. The kit of parts of claim 1, wherein said light emitting device comprises a handle, a light output head, and at least one light source contained in said light output head.

3. The kit of parts of claim 2, wherein the light output head is configured as an angular, oval, or rounded head, as a tray or as a bite splint.

4. The kit of parts of claim 2, wherein the light output head is configured to emote the light within all directions to the oral cavity.

5. The kit of parts of any one of claims 2 to 4, wherein the light output head contains multiple light sources.

6. The kit of parts of any one of claims 2 to 4, wherein the light output head further comprises a diffuser.

7. The kit of parts of any one of claims 1 to 6, wherein the light is produced by one or several light-emitting diodes.

8. The kit of parts of any one of claims 2 to 4, or 6, wherein light source emits blue light.

9. The kit of parts of claim 8, wherein the blue light is at a wavelength in the range of 400 nm to 500 nm.

10. The kit of parts of any one of claims 1 to 9, wherein the oral composition comprising curcumin, curcuminoids, or a derivative thereof is a fluid, a paste, or a gel.

11. The kit of parts of any one of claims 1 to 10, wherein the concentration of curcumin, or curcuminoid, curcumin derivative, or hypericin ranges from 0.01 to 10 mg/mL, or from 0.05 to 5 mg/mL, or from 0.1 to 3 mg/mL.

12. The kit of parts of any of claims 1 to 11, wherein the curcumin, or the curcuminoid, or the derivative thereof is colorless.

13. A dental hygiene composition for photo disinfection of the oral cavity, said composition comprising curcumin, curcuminoids, a curcumin derivative, or hypericin, wherein said curcumin, said curcuminoids, said curcumin derivative, or said hypericin is associated with polyvinylpyrrolidone.

14. The dental hygiene composition according to claim 13, wherein the concentration of said curcumin, curcuminoids, curcumin derivative, or hypericin ranges from 0.01 to 10 mg/mL, or from 0.05 to 5 mg/mL, or from 0.1 to 3 mg/mL.

15. The dental hygiene composition according to claim 13 or 14, wherein the curcumin, the curcuminoid, or the curcumin derivative is colorless, and/or wherein said composition is a fluid, a paste, or a gel.

## Patentansprüche

1. Teilesatz für ein Mundpflegegerät zur Photodesinfektion der Mundhöhle, wobei der Teilesatz Folgendes umfasst:
a. eine Lichtemissionsvorrichtung, und
b. eine orale Zusammensetzung, die Curcumin, ein Curcuminoid, ein Curcumin-Derivat oder Hypericin umfasst,
wobei das Curcumin, das Curcuminoid, das Curcumin-Derivat oder das Hypericin mit Polyvinylpyrrolidon assoziiert ist.

2. Teilesatz nach Anspruch 1, wobei die lichtemittierende Vorrichtung einen Griff, einen Lichtausgabekopf und mindestens eine Lichtquelle, die in dem Lichtausgabekopf enthalten ist, umfasst.

3. Teilesatz nach Anspruch 2, wobei der Lichtausgabekopf als ein eckiger, ovaler oder abgerundeter Kopf, als eine Schale oder als eine Aufbissschiene ausgelegt ist.

4. Teilesatz nach Anspruch 2, wobei der Lichtausgabekopf dazu ausgelegt ist, das Licht innerhalb aller Richtungen zu der Mundhöhle auszugeben.

5. Teilesatz nach einem der Ansprüche 2 bis 4, wobei der Lichtausgabekopf mehrere Lichtquellen enthält.

6. Teilesatz nach einem der Ansprüche 2 bis 4, wobei der Lichtausgabekopf ferner einen Diffusor umfasst.

7. Teilesatz nach einem der Ansprüche 1 bis 6, wobei das Licht durch eine oder mehrere Leuchtdioden erzeugt wird.

8. Teilesatz nach einem der Ansprüche 2 bis 4 oder 6, wobei die Lichtquelle blaues Licht emittiert.

9. Teilesatz nach Anspruch 8, wobei das blaue Licht eine Wellenlänge im Bereich von 400 nm bis 500 nm aufweist.

10. Teilesatz nach einem der Ansprüche 1 bis 9, wobei die orale Zusammensetzung, die Curcumin, Curcuminoide oder ein Derivat davon umfasst, ein Fluid, eine Paste oder ein Gel ist.

11. Teilesatz nach einem der Ansprüche 1 bis 10, wobei die Konzentration von Curcumin oder Curcuminoid, Curcumin-Derivat oder Hypericin im Bereich von 0,01 bis 10 mg/ml oder von 0,05 bis 5 mg/ml oder von 0,1 bis 3 mg/ml liegt.

12. Teilesatz nach einem der Ansprüche 1 bis 11, wobei das Curcumin oder das Curcuminoid oder das Derivat davon farblos ist.

13. Zahnpflegezusammensetzung zur Photodesinfektion der Mundhöhle, wobei die Zusammensetzung Curcumin, Curcuminoide, ein Curcumin-Derivat oder Hypericin umfasst, wobei das Curcumin, die Curcuminoide, das Curcumin-Derivat oder das Hypericin mit Polyvinylpyrrolidon assoziiert sind.

14. Zahnpflegezusammensetzung nach Anspruch 13, wobei die Konzentration des Curcumins, der Curcuminoide, des Curcumin-Derivats oder des Hypericins im Bereich von 0,01 bis 10 mg/ml oder von 0,05 bis 5 mg/ml oder von 0,1 bis 3 mg/ml liegt.

15. Zahnpflegezusammensetzung nach Anspruch 13 oder 14, wobei das Curcumin, das Curcuminoid oder das Curcumin-Derivat farblos ist und/oder
wobei die Zusammensetzung ein Fluid, eine Paste oder ein Gel ist.

## Revendications

1. Kit de pièces pour instrument de soins buccaux pour photo-désinfection de la cavité buccale, le kit de pièces comprenant :
a. un dispositif électroluminescent, et
b. une composition orale comprenant de la curcumine, un curcuminoïde, un dérivé de curcumine, ou de l'hypericine,
ladite curcumine, ledit curcuminoïde, ledit dérivé de curcumine ou ladite hypericine étant associé à la polyvinylpyrrolidone.

2. Kit de pièces selon la revendication 1, ledit dispositif électroluminescent comprenant une poignée, une tête électroluminescente, et au moins une source de lumière contenue dans ladite tête électroluminescente.

3. Kit de pièces selon la revendication 2, la tête électroluminescente étant configurée comme une tête angulaire, ovale ou arrondie, comme un plateau ou comme une attelle de morsure.

4. Kit de pièces selon la revendication 2, la tête électroluminescente étant configurée pour émettre la lumière dans toutes les directions vers la cavité buccale.

5. Kit de pièces selon l'une quelconque des revendications 2 à 4, la tête électroluminescente contenant de multiples sources de lumière.

6. Kit de pièces selon l'une quelconque des revendications 2 à 4, la tête électroluminescente comprenant en outre un diffuseur.

7. Kit de pièces selon l'une quelconque des revendications 1 à 6, la lumière étant produite par une ou plusieurs diodes électroluminescentes.

8. Kit de pièces selon l'une quelconque des revendications 2 à 4 ou 6, la source de lumière émettant une lumière bleue.

9. Kit de pièces selon la revendication 8, la lumière bleue étant à une longueur d'onde dans la plage de 400 nm à 500 nm.

10. Kit de pièces selon l'une quelconque des revendications 1 à 9, la composition orale comprenant de la curcumine, des curcuminoïdes ou un dérivé de ceux-ci étant un fluide, une pâte ou un gel.

11. Kit de pièces selon l'une quelconque des revendications 1 à 10, la concentration de curcumine, ou curcuminoïde, dérivé de curcumine, ou hypericine étant comprise entre 0,01 et 10 mg/ml, ou entre 0,05 et 5 mg/ml, ou entre 0,1 et 3 mg/ml.

12. Kit de pièces selon l'une quelconque des revendications 1 à 11, la curcumine, ou le curcuminoïde, ou le dérivé de celui-ci étant incolore.

13. Composition d'hygiène dentaire pour photo-désinfection de la cavité buccale, ladite composition comprenant de la curcumine, des curcuminoïdes, un dérivé de curcumine, ou de l'hypericine, ladite curcumine, lesdits curcuminoïdes, ledit dérivé de curcumine ou ladite hypericine étant associé à la polyvinylpyrrolidone.

14. Composition d'hygiène dentaire selon la revendication 13, la concentration desdits curcumine, curcuminoïdes, dérivé de curcumine ou hypericine étant comprise entre 0,01 et 10 mg/ml, ou entre 0,05 et 5 mg/ml, ou entre 0,1 et 3 mg/ml.

15. Composition d'hygiène dentaire selon la revendication 13 ou 14, la curcumine, le curcuminoïde ou le dérivé de curcumine étant incolore, et/ou ladite composition étant un fluide, une pâte ou un gel.
